# EUROPEAN PATENT APPLICATION

(11) **EP 0 558 112 A1**
(43) Date of publication of application: **01.09.1993**
(21) Application number: 93200311.4
(22) Date of filing: 05.02.1993
(51) Int. Cl.: C12P 7/64, C11B 3/00, C11C 3/00, C12M 1/40

(54) **Enzymic diglyceride removal**

(30) Priority: 25.02.1992 EP 92301542
(71) Applicant: UNILEVER N.V., NL-3000 DK Rotterdam (NL); UNILEVER PLC, London EC4P 4BQ (GB)
(72) Inventor: Moore, Harry, Unilever Research Colworth, Sharnbrook Bedford MK44 1LQ (GB); Moore, Stephen Raymond, Unilever Research Colworth, Sharnbrook Bedford MK44 1LQ (GB)
(74) Representative: Hartong, Richard Leroy

(57) **Abstract**

The invention concerns a process for the enzymic removal of diglycerides from glyceride mixtures. Herefore, the glyceride mixture is contacted with an enzyme specific for the conversion of diglycerides. This contact is performed in an aqueous emulsion of the diglycerides, wherein the droplet size of the dispersed phase is less than 100 µm.

## Description

At present, many methods for the preparation of triglycerides are known in the prior art. Among these, the directed enzymic interesterification methods are the most elegant ones for the preparation of triglycerides with a required specific distribution of the fatty acid residues over the triglyceride. Although in this directed, enzymic esterification process a minimum amount of water is used, i.e. just enough water to activate the enzyme, it is inevitable that some amount (2-10 wt.%) of diglyceride is formed. The presence of these diglycerides in the main product (= triglycerides) is very unfavourable as the diglycerides have an adverse effect on the product properties of the triglycerides.

A number of processes coping with this problem are disclosed in literature. These are focussed on the removal of diglycerides from mixtures with triglycerides, wherein an enzymic conversion of diglycerides is carried out by using an enzyme specific for the hydrolysis of diglycerides into glycerol and free fatty acids.

In JP 62/51997, e.g., a method for the improvement of fats is disclosed, wherein a mixture containing at least 70 wt.% of triglycerides and not less than 2 wt.% of diglycerides is contacted with an enzyme specific for partial glycerides in the presence of a small amount of water.

A similar process is disclosed in JP 62/61590. The hydrolysis of the partial glycerides is now followed by an esterification process using a 1,3-specific enzyme.

In JP 62/287 a similar process is disclosed by which the hydrolysis of monoglycerides and/or diglycerides is carried out by using the lipase produced by Penicillium cyclopium ATCC 34613.

Although these prior art processes perform rather well with respect to the level of diglycerides present in the end product, they have one main disadvantage : the reaction time is long because of a rather slow reaction rate. Although this could be overcome, at least partly, by using big reactors, the cost of investment involved will be high.

We have now found a new process by which the high cost of investment can be avoided and wherein the reaction rate is increased.

Therefore, our invention concerns a process for the enzymic removal of diglycerides from glyceride mixtures containing at least diglycerides and triglycerides by contacting the glyceride mixtures in an enzymic conversion zone with an aqueous solution of an enzyme specific for the conversion of diglycerides, wherein the contact of the aqueous enzyme solution and glyceride mixture is established in an emulsion of the glyceride mixture in the aqueous enzyme solution, the water content of the emulsion being 15-50 wt.% and the droplet size of the glycerides in the emulsion being less than 100 µm.

The above-mentioned process is new and inventive over a process for the transesterification of a triglyceride with a fatty acid in the presence of a lipase, wherein the transesterification is carried out in a thermodynamically-stable solution, such as a micro emulsion, which requires the use of a hydrophobic part, a surface-active part and water, as described in EP 237,092. According to this prior art process, esterification is aimed at, for which only a small amount of water needs to be present. Moreover, the use of an enzyme for the specific removal of diglycerides is not disclosed in this document. Furthermore, in this known process it is essential to use surfactants, which might be difficult to remove from the end product.

Very acceptable results are achieved, in particular with respect to reaction times, by carrying out our process as a counter-current process wherein fresh glyceride mixture is fed to one end of the conversion zone and the enzyme solution is fed to the opposite end of the conversion zone.

The conversion zone used can be a contactor, whereby the contactor can be of any known type, such as a rotating disc contactor, a multi-stage sieve tray column, a packed column or mixer/settler devices.

It is important to control the droplet size of the glyceride mixture in the emulsion. A droplet size of less than 100 µm already leads to favourable results. It is, however, preferred to use smaller droplet sizes. The preferred droplet sizes are therefore less than 50 µm, the mean diameter of the droplets being 5-20 µm.

In order to achieve such a mean diameter, it is preferred that the oil and water phase be fed into a high-shear mixer of the Silverson type.

A very convenient procedure is obtained when the mixing device is part of an enzymic conversion zone.

In the above-mentioned processes, the contact time of the reactants in the conversion zone can be kept at 1 minute to 60 minutes, in particular at 5-30 minutes.

The crude reaction product from the conversion zone is normally separated into a glyceride phase and into an aqueous phase rich in controllable levels of glycerol and containing part of the enzymes. Any type of separator can be used; however, centrifuges are preferred.

The level of glycerol in the aqueous phase can be controlled by taking samples and determining the reaction parameters, such as ratio of components, reaction time, droplet size in the emulsion etc., depending on the glycerol level of the aqueous stream.

In a very convenient process, the aqueous phase containing part of the enzymes is recirculated to the enzymic conversion zone. In this way, the enzyme solution is used in a very efficient way. However, in order to maintain a desired activity in the enzyme solution, it will be necessary to add fresh, make-up enzyme solution to the recycle stream of aqueous enzyme. Suitable make-up enzyme is added in such an amount that the concentration of enzyme in the recirculated enzyme solution is at least 0.05 wt.%, calculated on the oil.

In order to avoid the glycerol concentration building up to undesirably high levels, at least part of the aqueous phase, rich in glycerol, is removed as purge from the system.

The glyceride phase obtained in the process can be washed with water, whereupon a triglyceride-rich product, from which most of the diglycerides have been removed, is separated from an aqueous phase containing at least part of the enzymes, which aqueous phase is at least partly recirculated.

Although the recycle enzyme solution can be used in a manner co-current to the process stream, it is also possible to use the recycle stream in a way that is counter-current to the process stream.

This last-mentioned process is preferably applied when several mixer/conversion devices and several separators are used. The aqueous stream(s) containing the enzymes withdrawn from the separator(s) are then used in a manner counter-current to the process stream. The processes that can be carried out are illustrated by the processes according to the different flow sheets.

In the Figures :
- - Fig. 1: illustrates a counter-current process;
- - Fig. 2: illustrates a co-current process with recycle;
- - Fig. 3: as Fig. 2, but including a wash step;
- - Fig. 4: illustrates a counter-current process with recycle;
- - Fig. 5: illustrates an alternative counter-current process with recycle.

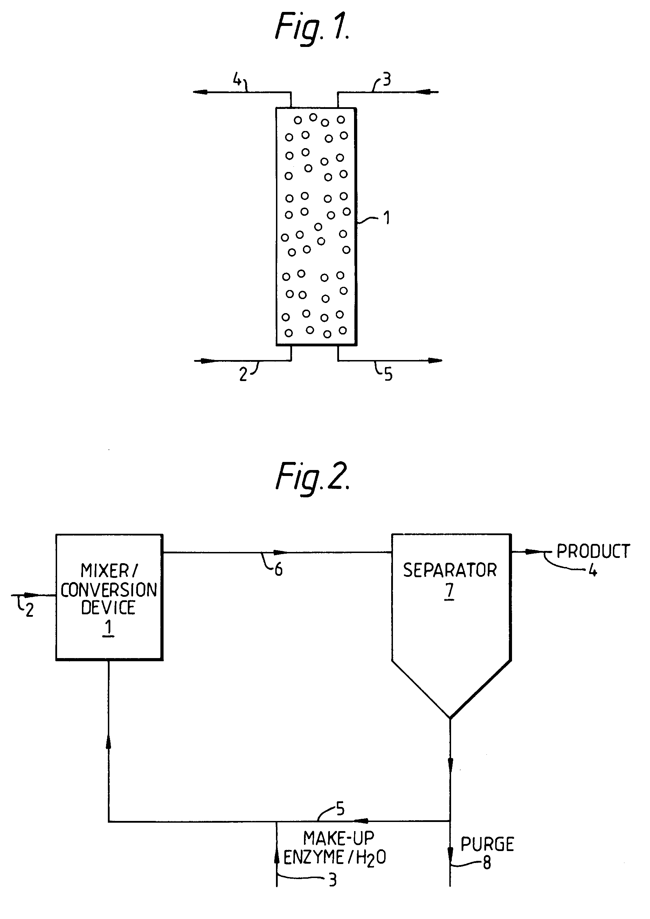

According to the process of Fig. 1, an enzymic conversion zone (1) is fed with a glyceride mixture (2) in a way counter-current to an aqueous enzyme solution (3). The enzymic conversion zone (1) can be a rotating disc contactor, a multi-stage sieve-tray column, a packed column or mixer/settler devices, the only requirement being that a droplet size of the glyceride mixture in the water phase of less than 100 µm can be achieved. From the conversion zone (1), a product consisting of triglycerides and free fatty acids, but with a reduced level of diglycerides is removed via (4), while an aqueous enzyme solution, also containing glycerol, is removed via (5).

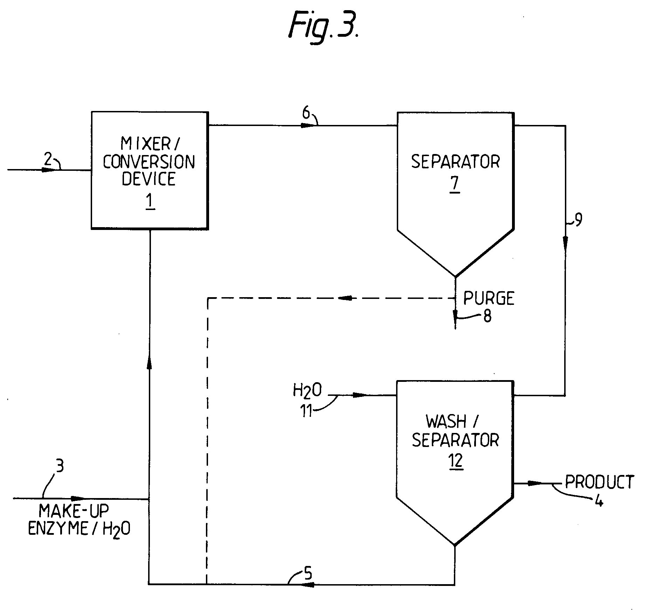

According to the processes of Fig. 2 and 3, a triglyceride oil containing 1-15 wt.% of diglycerides is mixed with an aqueous enzyme solution (0.02-1 wt.% of enzymes specific for the hydrolysis of diglycerides) in a mixer (1). An emulsion of fat in water with a mean droplet size of about 15 µm is obtained. This emulsion is led to an enzymic conversion zone which is part of the mixing device. The throughput through the conversion zone is chosen in order to obtain a residence time of about 15 minutes.

The crude reaction product (6) from (1) is led to a separator (7), wherein the product is split into an aqueous stream (8) high in glycerol and a triglyceride stream (4) or (9), which latter can be led to a wash/separator (12), where it is washed with water (11).

From wash/separator (12) a product stream (4) consisting of the desired triglycerides is obtained. An aqueous stream (5) containing the enzyme is led to a mixer where it is mixed with make-up enzyme (3), whereupon the mixture is recirculated to mixer/conversion zone (1).

The aqueous stream high in glycerol (8) can be removed as purge stream; however, it is, of course, also possible to feed part of this purge stream (8) into the make-up zone in order to re-use the enzymes present in this aqueous stream.

However, it will always be necessary to remove part of stream (8) as a purge; otherwise the level of glycerol will become too high to obtain acceptable results.

In the above-mentioned process, 2-8 parts of glyceride mixture are in general applied for 1 part of enzyme solution. This ratio means therefore that considerable amounts of water are present in the reaction mixture.

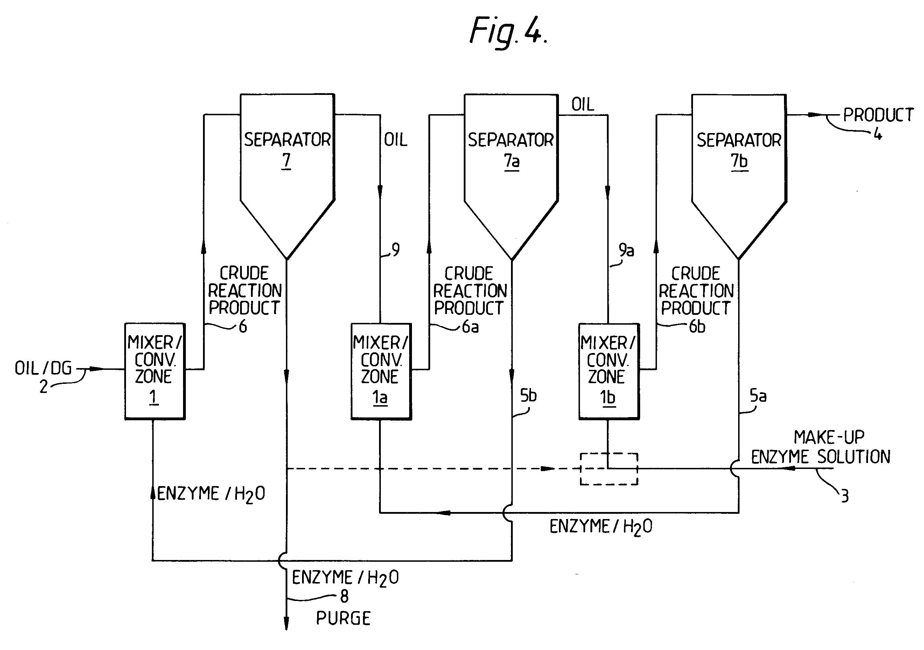

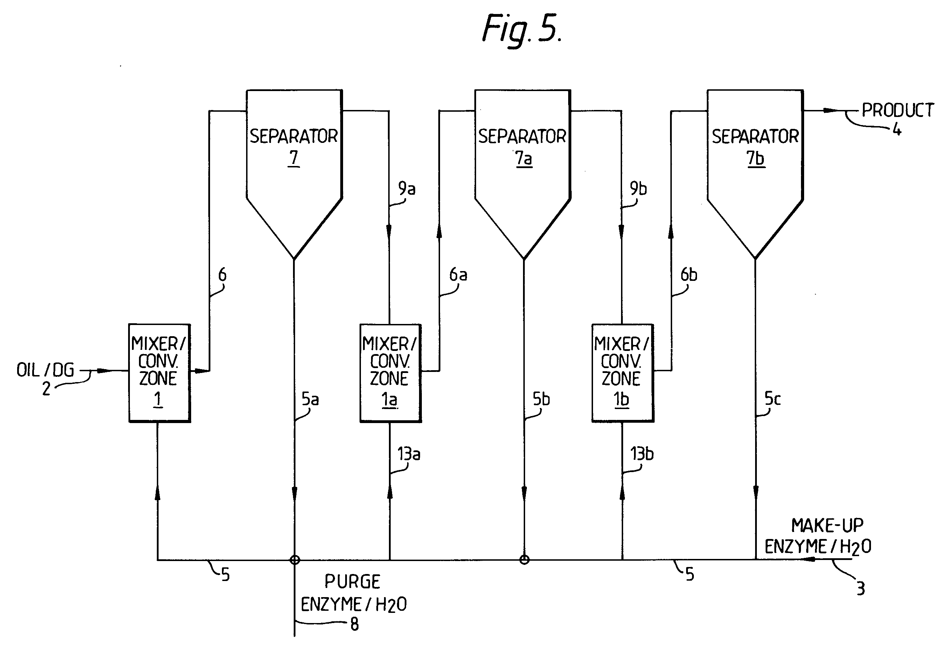

In Fig. 4 and 5, multi-stage processes are represented wherein the recycle aqueous enzyme solution is used in a manner counter-current to the process stream.

According to Fig. 4, three mixer/conversion zones (1, 1a and 1b) are used. Each conversion zone is followed by a separator (7, 7a and 7b).

The starting triglyceride mixture (2) is mixed with recycle enzyme solution (5b) from separator (7a) and is converted in zone (1). The crude reaction product is led via (6) to separator (7). From separator (7), a triglyceride stream (9) and an enzyme solution (8) as purge stream are obtained. The glyceride stream (9) is mixed with recycle enzyme solution (5a) from separator (7b) in mixer/conversion zone (1a). The crude reaction product is led via (6a) to separator (7a) and separated into a glyceride stream (9a) and the recycle enzyme solution (5b). The glyceride stream (9a) is mixed with fresh, make-up enzyme (3) in mixer/conversion zone (1b). The crude material (6b) is separated in separator (7b) into a product stream (4) and the recycle enzyme stream (5a). In this process also part of the purge (8) can be mixed with make-up enzyme solution (3), whereupon this mixture is led to zone (1b).

According to Fig. 5, a similar process is carried out. However, the enzymic solutions (5a, 5b and 5c) obtained in the different separators (7, 7a and 7b) are all led to a main recycle line (5), which is also fed with the make-up enzyme solution (3). Part of the recycle enzyme stream (5) is discarded as purge (8). This is done after the recycle stream (5a) has been combined with the main recycle stream (5).

### EXAMPLE

The process is carried out, using the flow sheet of Fig. 2. In this process, a mixture of 1840 kg of triglycerides and 160 kg of diglycerides is mixed per hour in a Silverson mixing device with a water phase comprising 836 kg of water, 160 kg of glycerol and 4 kg of enzyme (Amono-G), resulting in an emulsion with a droplet size of about 25 µm.

This emulsion is converted during a residence time of 15 minutes in the conversion zone.

The crude reaction mixture containing 2 wt.% of diglycerides is separated in centrifuge (7). 1990 Kg/h of a glyceride product (4) containing 2 wt.% of diglycerides and 5 wt.% of free fatty acids are obtained via line (4).

A water phase comprising 829 kg of water, 178 kg of glycerol and 4.0 kg of enzyme is removed from the centrifuge.

From this water phase, 101 kg/h is discarded as a purge via line (8).

The remainder is mixed with 90 kg of make-up enzyme solution (containing 0.4 kg of enzyme) and the enzyme solution is recirculated to mixer (1).

## Claims

1. Process for the enzymic removal of diglycerides from glyceride mixtures containing at least diglycerides and triglycerides by contacting the glyceride mixtures in an enzymic conversion zone with an aqueous solution of an enzyme specific for the conversion of diglycerides, characterized by the fact that the contact of the aqueous enzyme solution and glyceride mixture is established in an emulsion of the glyceride mixture in the aqueous enzyme solution, the water content of the emulsion being 15-50 wt.%, and the droplet size of the glycerides in the emulsion being less than 100 µm.

2. Process according to Claim 1, wherein the contact is established as a counter-current process, wherein fresh glyceride mixture is fed to one end of the conversion zone and the enzyme solution is fed to the opposide end of the conversion zone.

3. Process according to Claim 2, wherein the conversion zone is a contactor such as a rotating disc contactor, a multi-stage sieve tray column, a packed column, or mixer/settler devices.

4. Process according to Claim 1, wherein the droplet size of the glyceride mixture in the emulsion is less than 50 µm.

5. Process according to Claim 4, wherein the mean droplet size of the glyceride mixture is 5-20 µm.

6. Process according to Claim 1, wherein the emulsion is obtained by mixing of the glyceride and water phases in a high-shear mixer of the Silverson type.

7. Process according to Claim 6, wherein the mixing device is part of an enzymic conversion zone.

8. Process according to Claim 1 or 7, wherein the contact time of the reaction components in the conversion zone is kept between 1 and 60 minutes.

9. Process according to Claim 6, wherein the crude reaction product from the conversion zone is separated into a glyceride phase and into an aqueous phase rich in controllable levels of glycerol and containing part of the enzymes.

10. Process according to Claim 9, wherein the aqueous phase containing part of the enzymes is recirculated to the enzymic conversion zone.

11. Process according to Claim 10, wherein make-up enzyme is added to the aqueous phase containing the enzymes in order to increase the concentration of enzyme to the desired concentration of at least 0.05 wt.%, calculated on the oil, which enzyme solution is recirculated to the conversion zone.

12. Process according to Claims 1-11, wherein at least part of the aqueous phase rich in glycerol is removed as purge from the system.

13. Process according to Claim 9, wherein the glcyeride phase is washed with water, whereupon a product rich in triglycerides, is separated from an aqueous phase containing part of the enzymes, which aqueous phase is at least partly recirculated.

14. Process according to Claims 1, 10, 11 and 13, wherein the recycle stream is added in a manner co-current with the process stream.

15. Process according to Claims 1, 10, 11 and 13, wherein several subsequent mixer/conversion devices and several separators are used, the aqueous stream(s), containing the enzymes, withdrawn from the separator(s), being used in a manner counter-current to the process stream.
